Europäisches Patentamt

European Patent Office

Office européen des brevets

(19)

(11) Publication number: **0 096 514**

**A2**

# (12) EUROPEAN PATENT APPLICATION

(21) Application number: **83303040.6**

(22) Date of filing: **26.05.83**

(51) Int. Cl.³: **G 01 N 33/00**
**G 01 N 27/16**

(30) Priority: **05.06.82 GB 8216444**

(43) Date of publication of application:
**21.12.83 Bulletin 83/51**

(84) Designated Contracting States:
**DE FR GB IT SE**

(71) Applicant: **BL TECHNOLOGY LIMITED**
**35-38 Portman Square**
**London W1H OHQ(GB)**

(72) Inventor: **Parker, Peter Harry**
**203 Bromsgrove Road**
**Redditch Worcestershire(GB)**

(74) Representative: **Waters, Jeffrey**
**ARG Patent Department Cowley Body Plant**
**Cowley Oxford OX4 5NL(GB)**

(54) Detecting combustible or combustion-supporting constituents in exhaust gas from an internal combustion engine.

(57) A method and apparatus are provided for measuring air/fuel ratio for a fuel mixture (particularly for a spark ignition engine). A flow path is provided, for fuel mixture, which contains zones whereby either fuel can be added to lean mixtures or air can be added to rich ones (such additions being termed doping). In a subsequent reaction zone the temperature of, or current through, a conductor is maintained constant. The regulator needed to maintain the constant state enables the point of maximum heat release on combustion of a doped or undoped mixture near the conductor to be determined. A unique relationship between the ratio and such heat release enable the ratio to be determined.

FIG. 1

SOLENOID VALVE CONDITIONS

| VALUE | | 18,19 | 12,23 |
|---|---|---|---|
| RICH | DIRECT | ON | OFF |
| RICH | DOPED | ON | ON |
| LEAN | DIRECT | ON | OFF |
| LEAN | DOPED | OFF | OFF |

ALL VALVES SHOWN OFF

EP 0 096 514 A2

Croydon Printing Company Ltd

## DETECTING COMBUSTIBLE OR COMBUSTION-SUPPORTING CONSTITUENTS IN EXHAUST GAS FROM AN INTERNAL COMBUSTION ENGINE

This invention relates to apparatus for, and a method of, detecting combustible or combustion-supporting consitituents in exhaust gas from an internal combustion engine.

To achieve efficient operation with such engines, the optimum air/fuel ratio depends on various factors such as load, speed, water temperature etc, and may be lean (where the fuel proportion is low) or rich (where the fuel proportion is high) in comparison with a stoichiometric mixture. Various proposals have been put forward for detecting combustible or combustion-supporting constituents in the exhaust gas in order to measure the air/fuel ratio of the mixture entering the cylinders with the object of controlling the air/fuel ratio in a feed-back mode.

United Kingdom Patent application number 2 013 892 proposes an arrangement where exhaust gas is divided into two streams, one of which is saturated with whichever of a combustible or combustion-supporting material it is deficient in. Each of the two streams impinges on a hot wire sensor, whose resistance changes with temperature and the surface of which acts as a catalyst. The temperature difference measured between the streams is used to estimate the quantity of combustible or combustion-supporting constituents in the exhaust gas. It has been proposed in European Patent application number 34 013 to modify this proposal by using one sensor and alternately impinging the saturated and untreated streams onto it.

The invention provides apparatus for detecting combustible or combustion-supporting constituents in exhaust gas from an internal combustion engine comprising: a conductor the resistance of which varies with temperature and the surface of which acts as a catalyst; means for alternately impinging onto the conductor exhaust gas doped with whichever of a combustible or combustion-supporting material it is deficient in, and undoped exhaust gas; and means for repeatedly sampling the current and/or voltage in the conductor when doped exhaust gas impinges on it and obtaining values corresponding to maximum heat release, and for comparing those values with the values of the current and/or voltage in the conductor when undoped exhaust gas impinges.

The invention also provides a method of detecting combustible or combustion-supporting constituents in exhaust gas from an internal combustion engine comprising: alternately impinging on to a conductor exhaust gas doped with whichever of a combustible or combustion-supporting material it is deficient in, and undoped exhaust gas, the conductor having a resistance which varies with temperature and a surface which acts as a catalyst; repeatedly sampling the current and/or voltage in the conductor when doped exhaust gas impinges on it; obtaining values of current and/or voltage corresponding to maximum heat release; and comparing those values with the values of current and/or voltage in the conductor when undoped exhaust gas impinges.

The applicants have discovered that, in the lean region, the heat release obtained by saturating the exhaust gas with fuel as in the prior proposals is not directly related to the quantity of oxygen present in the gas but that the peak heat release within the period for which the doped gas impinges on the conductor, does give a measure of the quantity of oxygen present.

It is believed that the reason for this is that the heat release when doped exhaust gas impinges on the sensor rises as the amount of fuel rises to the stoichiometric quantity but then falls as the amount of fuel increases above that stoichiometric quantity. Different values of heat release are obtained depending on the degree of saturation. In contrast it has been found that the peak heat release within the period for which doped exhaust gas impinges on the sensor does give a measure of the stoichiometric amount of fuel needed to react the oxygen present, and hence does give a measure of that amount of oxygen.

It will be appreciated that the instantaneous heat release during each period for which doped fuel impinges on the sensor varies, starting from the beginning of the period when the amount of doping is less than stoichiometric to the end when the amount is in excess of stoichiometric.

There may be provided means for varying the voltage across the conductor to maintain a constant current in it. Preferably however there is provided means for varying the electrical power dissipated in the conductor to maintain the mean temperature of the conductor constant.

The conductor may be located in a passage connected at one end to the engine inlet manifold in order to draw exhaust gas in at the other end.

The exhaust gas may be doped in use by means spaced apart from the conductor and, immediately upstream of the conductor, the passage may be common to both doped and undoped flows. The arrangement ensures the steady increase of doping of the exhaust gas through the period for which doped exhaust gas impinges on the conductor.

The doping in the case of a lean mixture may be petrol but is preferably propane or butane.

It is preferable that at least the surface of the conductor is platinum or platinum/rhodium.

The periods for which doped and undoped exhaust gas impinge on the sensor may be from $\frac{1}{2}$ second to 10 seconds, preferably from 1 second to 5 seconds and the sampling rate may be from 1 microsecond to 100 milliseconds, preferably from 0.1 milliseconds to 10 milliseconds.

Apparatus for and a method of detecting combustible or combustion-supporting constituents in exhaust gas from a spark ignition engine will now be described, by way of example, with reference to the accompanying drawings, in which:

Figure 1 is a diagrammatic view of a first form of apparatus;

Figures 2 and 3 are graphs (for lean and rich mixtures respectively) of the relationship between heat output and the amount of doping needing to be added to the sample for a range of air to fuel ratios;

Figures 4 and 5 are graphs (for lean and rich mixtures respectively) of the difference in current needed to maintain constant temperature of the conductor with doped or undoped flow for various air/fuel ratios in the ideal steady state condition and with 2 second switching using the 'peak capture' method of the invention; and

Figure 6 is a diagrammatic representation of a second form of apparatus according to the invention.

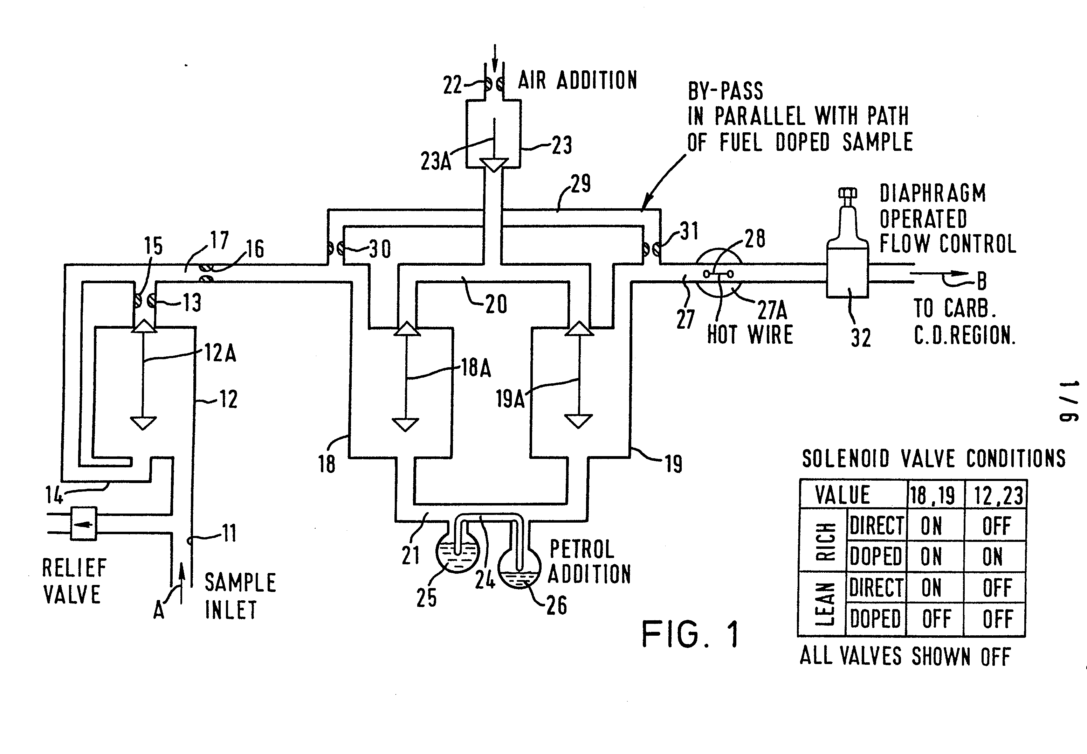

Referring to Figure 1, sample exhaust gas is caused to flow in the direction of arrow A along duct 11 to the upstream side of solenoid valve 12 which has opening and closing shuttle 12A. The valve has two outlets 13, 14. Outlet 13 incorporates a metering orifice 15. The two outlets 13 and 14 join upstream of orifice 16 to form a common duct 17. The pair of valves 18, 19 with shuttles respectively 18a, 19a are coupled by ducts 20, 21. Duct 20 is enabled to receive a supply of air through metering orifice 22 by way of valve 23 having a shuttle 23a. · Duct 21 enables petrol or other evaporative fuel to be released as a vapour into gas flowing along the duct 21 by way of a wick 24 which is fed liquid fuel by way of reservoirs 25, 26. Alternatively propane could be used using a valve similar to the valve 23. Passage 27 is coupled to the constant depression region of an SU (registered trade mark) carburettor feeding the inlet manifold of an internal combustion engine for a car. The resulting pressure drop established flow through the sensor in the direction of A and B. The passage 27 contains a reaction area 27a in which is disposed a platinum or platinum/rhodium conductor 28. The passage of current through this conductor is regulated by a conventional electric circuit under the control of a microprocessor to enable the heat of any reaction occuring in the vicinity of the wire 28 to be measured to establish the difference in electrical power required between a "doped" and "undoped" flow of combustible mixture. Flow along passage 27 is regulated by diaphragm valve 32. The valves 18, 19 are bypassed by a passage 29 containing metering orifices 30, 31. The passage 29 enables the exhaust gas flow which is passed along duct 21 and "doped" with petrol evaporated from wick 24 to be diluted with undoped exhaust gas to retard a build up of unburnt fuel. The wire 28 acts to measure the heat released by the chemical reactions on the wire. In the lean region these are between engine exhaust gas containing oxygen and added fuel, which can be any hydrocarbon. In the rich region the reactions are between engine exhaust gas containing carbon monoxide and the oxygen in the added air. The heat of reaction can be found by considering the total enthalpy of all the consitiuents before and after the reaction. This has been done for a series of engine exhaust air/fuel ratio values and various rates of air or fuel addition.

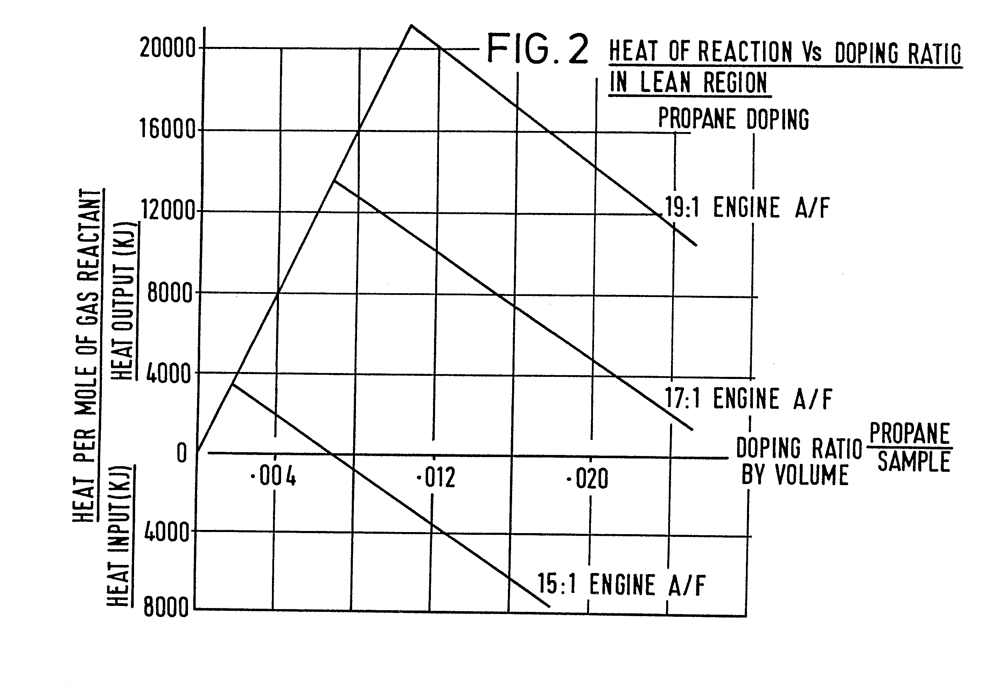

Figure 2 shows the results of the calculation for the lean region using propane as the doping fuel.

As the amount of propane doping is increased from zero at a given engine air/fuel ratio the heat of reaction increases up to a maximum. This maximum occurs when there is just sufficient propane to burn all the oxygen in the exhaust sample. Any increase in the doping beyond this causes the heat of reaction to reduce due to endothermic reactions, balancing some of the heat from the main exothermic reaction. If sufficient propane is added the heat output becomes negative, that is, heat is absorbed. The results shown in Figure 2 are supported by the observed behaviour of the sensor. They also explain non-linear calibrations obtained if the engine air/fuel ratio is varied for a fixed wick exposure area which corresponds to an approximately constant flow of liquid fuel for doping.

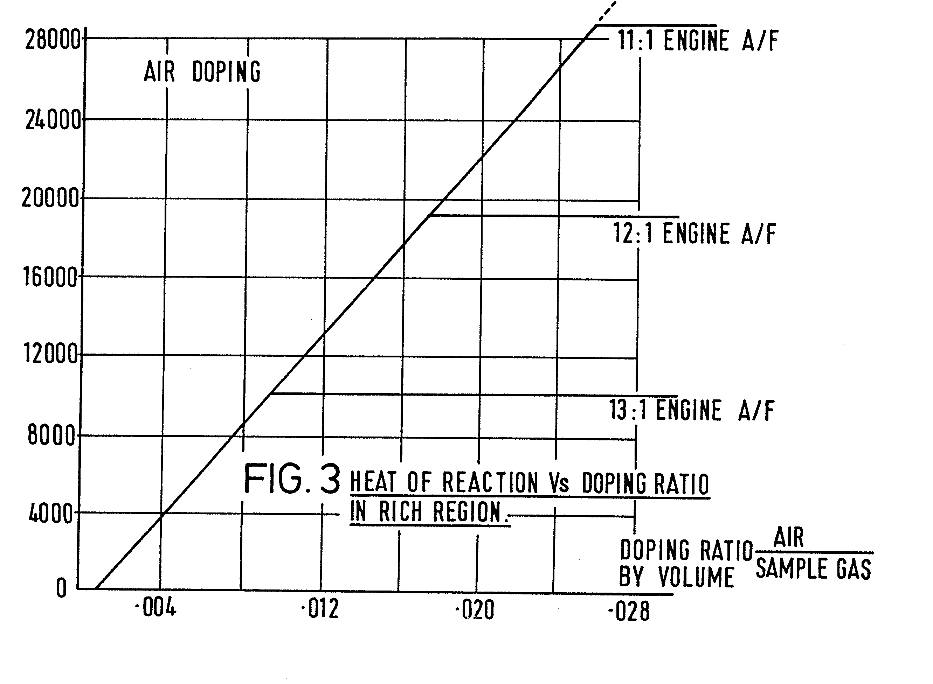

Figure 3 shows the results of calculation for the rich region where the behaviour is different. There is no change in the chemical reaction as the doping is increased above that required for peak heat release in contrast to the behaviour in the lean region described in the last paragraph. As the amount of air doping is increased at a given engine air/fuel ratio the heat of reaction increases until there is just sufficient oxygen to combine with all the carbon monoxide. After that a plateau is reached and extra air does not affect the heat release. The behaviour of the sensor in operation supports this theory. However, the output of the actual sensor gradually reduces as the amount of air is increased above that required to burn all the carbon monoxide because the dilution causes a lower carbon monoxide concentration in the gas diffusing onto the wire.

Sensor operation in the lean region involves the use of fuel addition by means of wick 24 (Figure 1). The wick is arranged to feed more than sufficient fuel to cover the leanest engine air/fuel ratio of interest, say 20:1. When valves 18, 19 are switched to their undoped positions the fuel concentration in the stream to the hot wire is zero. At some time after switching to the doped position the fuel concentration in the stream to the hot wire will be more than sufficient to give maximum heat release at 20:1 air/fuel ratio. Thus at some time between switching to the doped position and reaching this excess fuel condition the fuel concentration is momentarily correct for maximum heat release at the particular air/fuel ratio of the sampled flow. If the varying output of the sensor is recorded and the value corresponding to maximum heat release selected this accurately represents the sensor output for the particular air/fuel ratio. This process defined as "peak capture". To do this two methods have been used. In the first the sensor output is continually scanned at frequent intervals, in a particular example every 1.44 ms, in the period after switching by the microprocessor and the value corresponding to maximum heat release selected numerically. The alternative is to use a conventional analogue circuit to record the maximum value of heat release attained in the period after switching. This is read by the microprocessor later.

For the rich region it is not essential to use the "peak capture" method since excess air does not greatly reduce the heat release. However, in order to allow for the small reduction due to dilution it has been found convenient to use the method in the rich as well as the lean region.

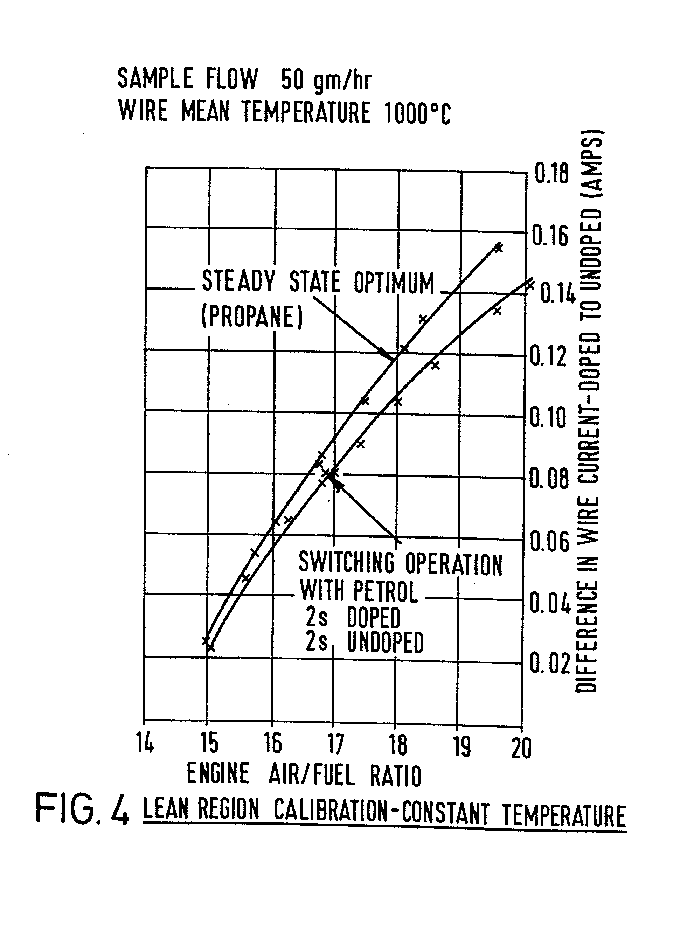

Figure 4 shows the lean region calibrations at a constant mean wire temperature of $1000^{o}C$. The curve labelled "steady state optimum" was obtained by adjusting the amount of propane for maximum heat release and the curve labelled "switching operation" was obtained by the "peak capture" method using petrol. This shows that the output obtained from the peak capture method corresponds closely to maximum heat release. The chosen wire temperature in the range 900 to $1000^{o}C$ only had a small effect of the calibration as long as it was kept constant.

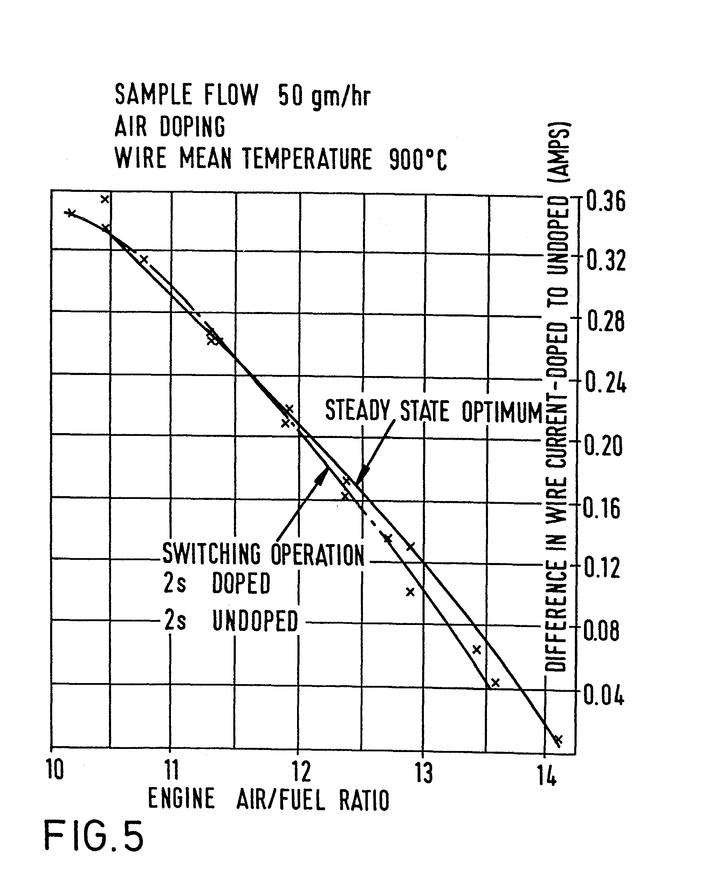

Figure 5 shows the calibrations in the rich region at $900^{o}C$ mean wire temperature. Again the curve labelled "steady state optimum" was obtained by adjusting the air addition for peak output and the curve labelled "switching operation" was obtained using the "peak capture" method and corresponds even more closely to maximum heat release. A further advantage of constant temperature operation over constant current operation in this region was the ability to run at an engine air/fuel ratio of 10.3:1 without overheating the wire.

The sensor has demonstrated an operating range from 10:1 air/fuel ratio on the rich side to 20:1 on the lean side with possible limited extensions if required. There is only a small indeterminate area either side of stoichiometric between the two ranges. The rich limit will cover warm-up fuel requirements at engine coolant water temperatures down to $-5^{\circ}$C. The lean limit is adequate to cover cruising air/fuel ratios likely to be used by conventional engines. It is difficult to estimate the absolute accuracy because only a limited number of wires have been tested. However, the experience suggests that an accuracy of $\pm$ 0.25 air/fuel ratio is possible with careful design. This is certainly acceptable for control of carburettors or other fuel systems in European applications.

When the invention is being used to provide the signal for feedback control of the air/fuel ratio the normal method of operation would involve specifying the desired air/fuel ratio from the known engine operating conditions. The appropriate doping, that is air or fuel, is therefore known. If the engine is running lean and air is added or if the engine is running rich and fuel is added the signal from the sensor which is the difference between wire current values for the doped and undoped samples, is zero. The microprocessor can therefore switch to the appropriate doping for the required air/fuel ratio and identify whether the engine has to be richened or leaned to bring the air/fuel ratio towards the target. It can then adjust the engine fuel system, for example the carburettor, as appropriate to gradually approach the required air/fuel ratio. When it is necessary to establish an unknown prevailing air/fuel ratio the presence of a zero signal from the sensor indicates that the wrong doping may be in use. The microprocessor can then control the necessary change. If this sensor output is zero and remains zero when the doping is changed from air to fuel or fuel to air then the air/fuel ratio lies in the relatively small indeterminate area on either side of stoichiometric between the two ranges.

It should be added that any unreacted combination of combustible and combustion-supporting constituents in the exhaust will be reacted on the hot wire in the undoped period. During that period, instead of measuring the peak value of the voltage or current in the wire, the voltage or current is averaged over the undoped period.

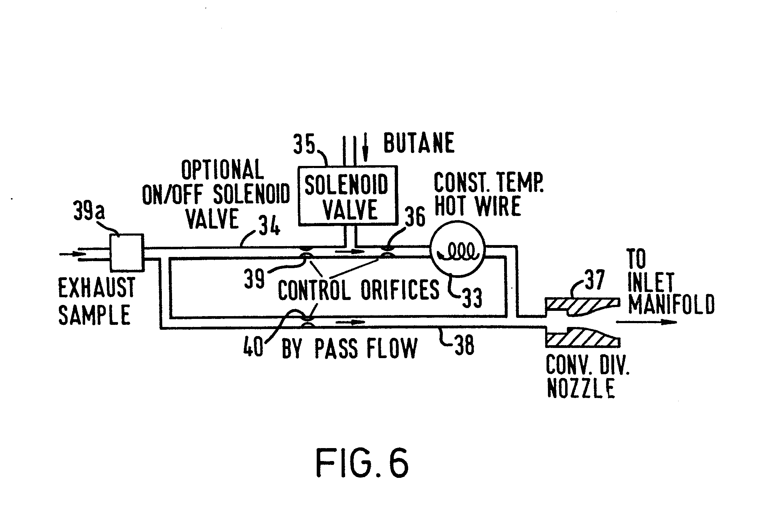

Referring to Figure 6, the second apparatus has a hot wire sensor 33 similar to the sensor 27a of the first apparatus on to which impinges alternately a sample of exhaust gas drawn along passage 34 and a sample of exhaust gas drawn along passage 34 but doped by means of butane controlled by solenoid valve 35. When the valve is open doped exhaust gas impinges. When the valve is closed undoped exhaust gas impinges. Orifice 36 ensures mixing of the butane with the exhaust gas.

The apparatus is connected to the inlet manifold via a convergent divergent nozzle 37 the surface of which is to keep the flow rate through the hot wire sensor 33 substantially constant over a range of different manifold depressions. The bypass flow 38 is provided to permit the use of a larger convergent divergent nozzle 37. Orifices 39 and 40 control the relative flows along passages 34 and 38. An on-off solenoid valve 39a may be provided in order to switch the apparatus off. The apparatus may be used as a periodic, that is, about once per day lean reference for checking and adjusting the fuel system's calibration. It need not be used for continuous feed-back control, although it could be if desired.

The sensor fuel is butane and is carried in a relatively small container.

The valve 35 opens and closes at two second intervals. The doped period is measured described by the first apparatus by sampling at 1.44 millisecond intervals, and the measurement for the undoped period is preduced by averaging the signal from the hot wire sensor over the last quarter of a second of each undoped period.

The hot wire sensor 33 is used in the constant temperature mode. The current (i) through the hot wire sensor 33 is related to the chemical heat release (H) per unit length of the wire by the following equation:

$$\frac{i\text{ UNDOPED} - i\text{ DOPED}}{i\text{ UNDOPED}} \doteq \frac{H}{2\left(\frac{Q_i}{l} + h\pi D\,\Theta_{mean} + \sigma_R - e\right)}$$

where the first term in the denominator gives a measure of the conduction into the supports of a wire length l; the second term represents heat loss due to convection per unit length, h being a function of Reynold's number, D being the diameter of the wire and $\Theta_{mean}$ being the amount by which the mean temperature of the wire exceeds that of the surrounding sample gas; the third term represents the small radiation loss and the fourth term represents the small heat release from unreacted constituents. The first term is also small for a length of wire in the range 5 millimetres to 20 millimetres as in the present invention. H and h are the same function of Reynold's number with the result that the left hand side of the expression is approximately equal to a constant for a given air fuel ratio. This means that the left hand side of the expression is relatively insensitive to flow variations. A particular test showed no change of this quantity for flow rates which varied between 80 and 120 grams per hour. The left hand side of the equation is also a function of the air/ fuel ratio, and that is how that quantity is determined.

It will be noted that the apparatus is used in the lean region only.

CLAIMS

1 Apparatus for detecting combustible or combustion-supporting constituents in exhaust gas from an internal combustion engine comprising: a conductor the resistance of which varies with temperature and the surface of which acts as a catalyst; means for alternately impinging onto the conductor exhaust gas doped with whichever of a combustible or combustion-supporting material it is deficient in, and undoped exhaust gas; and means for repeatedly sampling the current and/or voltage in the conductor when doped exhaust gas impinges on it and obtaining values corresponding to maximum heat release, and for comparing those values with the values of the current and/or voltage in the conductor when undoped exhaust gas impinges.

2 Apparatus as claimed in claim 1, wherein there is provided means for varying the voltage across the conductor to maintain a constant current in it.

3 Apparatus as claimed in claim 1, wherein there is provided means for varying the electrical power dissipated in the conductor to maintain the mean temperature of the conductor constant.

0096514

4      Apparatus as claimed in any one of claims 1 to 3, wherein the conductor is located in a passage connected at one end to the engine inlet manifold in order to draw exhaust gas in at the other end.

5      Apparatus as claimed in claim 4, wherein the exhaust gas is doped in use by means spaced apart from the conductor and, immediately upstream of the conductor, the passage is common to both doped and undoped flow.

6      Apparatus as claimed in claim 5, wherein there is provided an orifice downstream of the doping means.

7      Apparatus substantially as hereinbefore described with reference to and as shown in the accompanying drawings.

8      A method of detecting combustible or combustion-supporting constituents in exhaust gas from an internal combustion engine comprising: alternately impinging on to a conductor exhaust gas doped with whichever of a combustible or combustion-supporting material it is deficient in, and undoped exhaust gas, the conductor having a resistance which varies with temperature and a surface which acts as a catalyst; repeatedly sampling the current and/or voltage in the conductor when doped exhaust gas impinges on it; obtaining values of current and/or voltage corresponding to maximum heat release;
and comparing those values with the values of current
and/or voltage in the conductor when undoped exhaust
gas impinges.

9    A method as claimed in claim 8, wherein the voltage
across the conductor is varied to maintain a constant
current in it.

10    A method as claimed in claim 8, wherein the electrical
power dissipated in the conductor is varied to maintain
the mean temperature of the conductor constant.

11    A method as claimed in any one of claims 8 to 10, wherein
the inlet manifold depression is used to draw exhaust
gas across the conductor.

12    A method as claimed in claim 11, wherein the exhaust
gas is doped at a position spaced apart from the conductor
and the alternate streams of doped and undoped gas pass
to the conductor via a common passage.

13    A method as claimed in claim 12, wherein the doped
flow passes through an orifice before reaching the
conductor.

14    A method substantially as hereinbefore described with reference to the accompanying drawings.

FIG. 1

**SOLENOID VALVE CONDITIONS**

| VALUE | | 18,19 | 12,23 |
|---|---|---|---|
| RICH | DIRECT | ON | OFF |
| | DOPED | ON | ON |
| LEAN | DIRECT | ON | OFF |
| | DOPED | OFF | OFF |

ALL VALVES SHOWN OFF

FIG. 2  HEAT OF REACTION Vs DOPING RATIO IN LEAN REGION

PROPANE DOPING

19:1 ENGINE A/F

17:1 ENGINE A/F

15:1 ENGINE A/F

HEAT PER MOLE OF GAS REACTANT

HEAT OUTPUT (KJ)

HEAT INPUT (KJ)

20000

16000

12000

8000

4000

0

4000

8000

·004    ·012    ·020

DOPING RATIO BY VOLUME    PROPANE / SAMPLE

2/6

0096514

FIG. 3 HEAT OF REACTION Vs DOPING RATIO IN RICH REGION.

3/6

0096514

SAMPLE FLOW  50 gm/hr
WIRE MEAN TEMPERATURE 1000°C

STEADY STATE OPTIMUM
(PROPANE)

SWITCHING OPERATION
WITH PETROL
2s  DOPED
2s  UNDOPED

DIFFERENCE IN WIRE CURRENT—DOPED TO UNDOPED (AMPS)

ENGINE  AIR/FUEL RATIO

FIG. 4 LEAN REGION  CALIBRATION-CONSTANT TEMPERATURE

FIG.5

FIG.6